(19) Europäisches Patentamt
European Patent Office
Office européen des brevets

(11) Publication number: **0 175 304**
**A2**

# (12) EUROPEAN PATENT APPLICATION

(21) Application number: 85111611.1

(22) Date of filing: 13.09.85

(51) Int. Cl.⁴: **C 07 C 131/00**, C 07 D 307/52, C 07 D 317/58, C 07 D 333/22, A 61 K 31/15

(30) Priority: 13.09.84 IT 2264684

(43) Date of publication of application: 26.03.86 Bulletin 86/13

(84) Designated Contracting States: AT BE CH DE FR GB LI LU NL SE

(71) Applicant: LABORATORI BALDACCI SPA, Via San Michele degli Scalzi 73, I-56010 Pisa (IT)

(72) Inventor: Macchia, Bruno, Via Lavagna 15, I-56100 Pisa (IT)

(74) Representative: Barz, Peter, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8, D-8000 München 40 (DE)

## (54) Compounds derived from beta-oximino-propionic acid having therapeutic activity, process for their preparation, and pharmaceutical compositions containing them.

(57) A new series of organic chemical compounds classifiable as derivatives of propionic acid substituted on beta with an iminoether group has antiinflammatory, analgesic, antipyretic, platelet aggregation inhibiting and cytostatic pharmacological properties such as to allow their therapeutic employment in all pathologies involved in phlogogenic, platelet hyperactivity and oncogenic processes.

0 175 304

# COMPOUNDS DERIVED FROM BETA-OXIMINO-PROPIONIC ACID HAVING THERAPEUTIC ACTIVITY, PROCESS FOR THEIR PREPARATION, AND PHARMACEUTICAL COMPOSITIONS CONTAINING THEM

The present invention relates to derivatives of propionic acid substituted on beta by an iminoether group having the general formula:

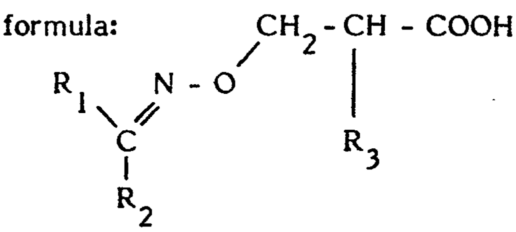

wherein $R_1$ and $R_2$ are a hydrogen atom or alkyl groups containing from 1 to 4 carbon atoms, straight or branched, variously substituted or unsubstituted aryls or heterocyclic groups, or a butylene or pentylene group, while $R_3$ is hydrogen or an alkyl group with up to two carbon atoms, and their nontoxic and pharmaceutically acceptable salts. When a geometrical isomerization around the double bond $>C=N-$ is possible the present invention extends to both possible isomer conformations.

The compounds of the present invention may be considered analogs of known nonsteroid antiinflammatory agents in which the aromatic ring is substituted by the moiety:

$>C=N-O-CH_2-$

On the basis of the present invention the iminoether group proved to be capable of generating a peculiar electron distribution which qualitatively keeps the pharmacological properties of the nonsteroid antiinflammatory analogs. The magnitude of the pharmacological effect is in some cases significantly superior and, surprisingly, half-life proves to be considerably increased. In fact, as will be seen below, one of the compounds of the invention,

contrary to the nonsteroid antiinflammatory analogs belonging to the phenylacetic acid family (e.g. the compound known as Diclofenac) has a half-life greater than 12 hours compared to approximately 2 hours for the corresponding phenylacetic acid used as a reference, making possible therapeutic use in a single daily dose instead of by repeated administrations at brief time intervals.

By appropriate chemical substitutions the synthesized compounds of the present invention correspond to the above structure in which the respective substituents are given for each of them in Table I.

TABLE I

| Compound | $R_1$ | $R_2$ | $R_3$ | MW | MP (BP) |
|---|---|---|---|---|---|
| 1 | | $CH_2(CH_2)_2$-$CH_2$ | H | 171 | 60-61°C |
| 2 | | $CH_2(CH_2)_3$-$CH_2$ | H | 185 | 38-39°C |
| 3 | $CH_3$ | -$CH_3$ | H | 145 | -104-106°C at 0.4mmHg |
| 4 | $CH_3$-$CH_2$-$CH_2$ | $CH_3$-$CH_2$-$CH_2$ | H | 201 | -123-124°C at 0.3 mmHg |
| 5 | $(CH_3)_2$-CH- | $(CH_3)_2$-CH | H | 201 | -109-110°C at 0.4 mmHg |
| 6 | $C_6H_5$ | $CH_3$ | H | 207 | 66-68°C |
| 7 | $C_6H_5$ | $C_6H_5$ | H | 269 | 102°C |
| 8 | $C_6H_5$ | H | H | 193 | 65-67°C |
| 9 | 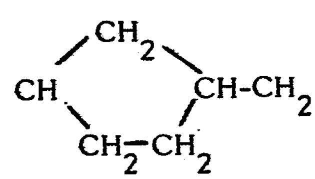 | | H | 187 | -175°C at 2 mmHg |
| 10 | 4-$CH_3O$-$C_6H_4$ | H | H | 233 | 80-82°C |
| 11 | 4-Cl-$C_6H_4$ | $H_3C$ | H | 242 | 84-86°C |
| 12 | 4-$CH_3O$-$C_6H_4$ | $H_3C$ | H | 237 | 103-107°C |

| | | | | | |
|---|---|---|---|---|---|
| 13 | 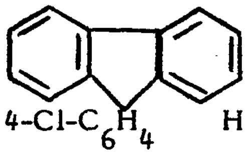 | | H | 267 | 127-129°C |
| 14 | 4-Cl-$C_6H_4$ | H | H | 228 | 126-128°C |
| 15 | 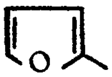 | H | H | 183 | 181-183°C |
| 16 | 2.5-$CH_3O$-$C_6H_3$ | H | H | 253 | 71-73°C |
| 17 | 2-Cl-$C_6H_4$ | H | H | 228 | 53-55°C |
| 18 | 3-Cl-$C_6H_4$ | H | H | 228 | 85-87°C |
| 19 | 3-$CH_3O$-$C_6H_4$ | H | H | 223 | 79-80°C |
| 20 | 3-$CH_3O$-$C_6H_4$ | H | H | 223 | 56-58°C |
| 21 | 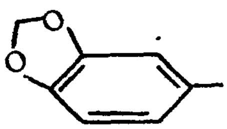 | H | H | 237 | 126-128°C |
| 22 | 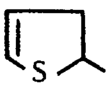 | H | H | 199 | 164-165°C |
| 23 | H | $C_6H_5$ | H | 193 | 161-163°C |
| 24 | $C_6H_5$ | H | -$CH_3$ | 207 | 170-174°C |
| 25 | $C_6H_5$ | H | -$C_2H_5$ | 222 | 122-124°C |

It is clear that within the limits of the abovementioned substitutions any other substitution with analogous groups which could be provided in theory in accordance with numerous hypothetical combinations would fall within the objects of the present invention.

It is evident on the basis of the present invention that any substitution of oximinether chains for benzene rings in known pharmacologically active structures makes it possible to retain the pharmacological properties of the reference structure.

This is shown by what was given above and by the results obtained from the substitutions made on other known pharmacologically active substances belonging to other therapeutical classes.

In fact substitution of activated oximinether chains for the

benzene ring in the (S)-6-fluoro-2-3-dihydrospiro (4H-1-benzopyran-4,4'-imidazolidin)-2',5' dione (sorbinil), a substance known as an inhibitor of the reductase aldose, and in penicillin G (benzyl-penicillin), an antibiotic widely used in the therapy of infections from gram-positive bacteria, makes it possible to keep qualitatively the same pharmacological profile.

The compounds object of the present invention are obtained in accordance with a process represented by the following general scheme:

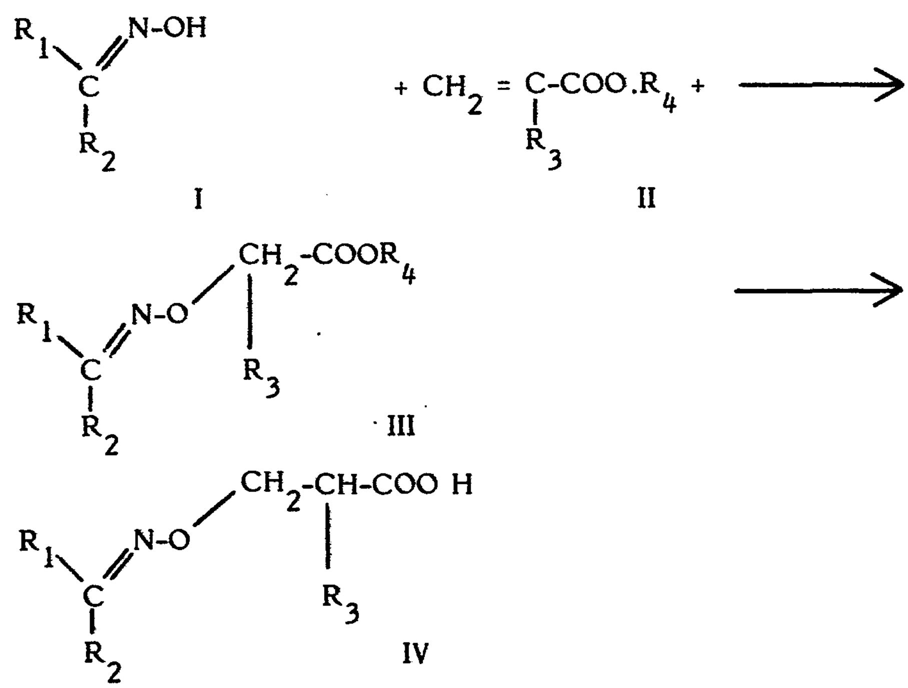

I

II

III

IV

wherein $R_1$, $R_2$ and $R_3$ have the aboveindicated meanings and $R_4$ is an alkyl.

The compounds are then obtained starting from the corresponding oximes (I) which are added to the appropriate alkyl acrylate, preferably ethyl acrylate, (II) in the presence of a catalytic quantity of KOH in accordance with processes well known to those skilled in the art, preferably in t-butyl alcohol, at a temperature of between 35°C and 60°C.

Thus are obtained the esters (III) which by saponification with KOH, preferably at room temperature in an ethanol solution, give the related acids (IV) which comprise the object of the present invention.

As an illustrative but nonlimiting example of the present invention the preparation technique for several compounds indicated in the general synthesis scheme is given.

Example 1

Preparation of the ethyl ester of beta-oximinopropionic acid III ($R_1=C_6H_5, R_2=R_3=H$).

To a solution of acetophenanoxime ($R_1=C_6H_5, R_2=H$) (28.0 g, 0.23 mol) in t-butyl alcohol (160 ml) is added a solution of 2N KOH in absolute ethanol (19 ml, 0.038 mol) and ethyl acrylate ($II:R_3=H$) (20.7 ml, 0.19 mol) and the resulting mixture is introduced into a one-necked flask with a ground glass stopper and stirred magnetically at 35°C for 48 hours. After evaporation of the solvent at reduced pressure the residue is taken up with $Et_2O$ and the organic phase is washed 3 times with $H_2O$, dried over anhydrous $MgSO_4$ and evaporated at reduced pressure. A crude oil is obtained which by distillation at reduced pressure gives III ($R_1=C_6H_5, R_2=R_3=H$) (15.5 g, 37% calculated on the ethyl acrylate):bp 79°C/0.5 mmHg.

Example 2

Preparation of beta-oximinopropionic acid (IV: $R_1$=$C_6H_5$,$R_2$=$R_3$=H; compound 8).

A solution of III ($R_1$=$C_6H_5$,$R_2$=$R_3$=H) (35.0 g, 0.158 mol) in ethanol (25 ml) is treated with an ethanolic solution of 2N KOH (94.5 ml, 0.19 mol) and left for 48 hours at room temperature. After evaporation at reduced pressure of the organic solvent the solid residue is taken up with $H_2O$ and the aqueous phase is washed twice with $Et_2O$ and then acidified to pH 1-2 with dilute HCl and extracted 3 times with ethyl ether.

The ethereal phase washed twice with $H_2O$ and dried over anhydrous $MgSO_4$ is evaporated at reduced pressure to give a solid raw product (15.0 g) which by crystallization from petroleum ether/ethyl ether gives compound 8 practically pure (11.2 g, 39%).

Example 3

Preparation of ethyl ester of beta-oximinopropionic acid III ($R_2$=$C_6H_5$,$R_1$=$R_3$=H).

In a one-necked flask with a ground glass stopper is introduced a solution of I ($R_1$=H, $R_2$=$C_6H_5$) (10.0 g, 0.083 mol) in t-butanol (25 ml), a solution of 2N KOH in absolute ethanol (6.9 ml, 0.014 mol) and ethyl acrylate (II:$R_3$=H) (7.5 ml, 0.069 mol); the resulting mixture is stirred magnetically at 60°C for 72 hours. After evaporation of the solvent at reduced pressure the residue is taken up with $Et_2O$ and the organic phase washed 3 times with $H_2O$, dried over anhydrous $MgSO_4$ and evaporated at reduced pressure. Practically pure III ($R_1$=H, $R_2$=$C_6H_5$, $R_3$=H) (7.0 g, 46% calculated on the ethyl acrylate) is obtained.

Example 4

Preparation of beta-oximinopropionic acid (IV:$R_2$=$C_6H_5$, $R_1$-

$=R_3=H$; compound 23).

A solution of III ($R_1=H$, $R_2=C_6H_5$, $R_3=H$) (7.0 g, 0.0316 mol) in ethanol (5 ml) is treated with an ethanolic solution of 2N KOH (19 ml, 0.038 mol) and left at room temperature for 96 hours. After evaporation of the organic solvent at reduced pressure the solid residue is taken up with $H_2O$ and the organic phase washed twice with ethyl ether and then acidified with dilute HCl to pH 5-6 and extracted three times with ethyl ether.

The organic phase washed twice with $H_2O$ and dried over anhydrous $MgSO_4$ is evaporated at reduced pressure to give a solid raw product (2.5 g) which by crystallization from ethyl ether-/petroleum ether gives compound 23 practically pure (1.83 g, 32%).

Example 5

Preparation of the ethyl ester of beta-oximinopropionic acid III ($R_1=R_2=(CH_3)_2CH,R_3=H$).

To a solution of diisopropyloxime ($R_1=R_2$ $(CH_3)_2$-CH) (36.8 g, 0.25 mol) in t-butyl alcohol (208 ml) is added a solution of 2N KOH in absolute ethanol (20.8 ml, 0.041 mol) and ethyl acrylate (II:$R_3$=H) (20.8 g, 0.20 mol). The resulting mixture is stirred mechanically for 48 hours at 36°C.

After evaporation of the solvent at reduced pressure the residue is taken up with $Et_2O$ and the organic phase washed 3 times with $H_2O$, dried over anhydrous $MgSO_4$ and evaporated at reduced pressure.

A raw oil is obtained which by distillation at reduced pressure gives III ($R_1=R_2=(CH_3)_2$-CH,$R_3$=H) (24.3 g, 51% calculated on the ethyl acrylate): bp 95°C/2.5 mmHg.

Example 6

Preparation of beta-oximinopropionic acid (IV:$R_1=R_2=(CH_3)_2$-

CH, $R_3$=H; compound 5).

A solution of III ($R_1$=$R_2$=$(CH_3)_2$-CH,$R_3$=H) (20.3 g, 0.084 mol) in ethanol (14 ml) is treated with an ethanolic solution of 2N KOH (53 ml, 0.10 mol) and left at room temperature for 48 hours. After evaporation of the organic solvent at reduced pressure the solid residue is taken up with $H_2O$ and the organic phase is washed twice with ethyl ether and then acidified with dilute HCl to pH 1-2 and extracted 3 times with ethyl ether.

The organic phase washed twice with $H_2O$ and dried over anhydrous $MgSO_4$ is evaporated at reduced pressure to give a raw oil which by distillation at reduced pressure gives compound 5 (100 g, 55%).

Example 7

Preparation of the ethyl ester of beta-oximinopropionic acid III ($R_1$=$C_6H_5$,$R_2$=$CH_3$,$R_3$=H).

To a solution of acetophenonoxime I ($R_1$=$C_6H_5$,$R_2$=$CH_3$,$R_3$=H) (27.5 g, 0.20 mol) in t-butyl alcohol (127 ml) is added a solution of 2N KOH in absolute alcohol (16.9 ml, 0.034 mol) and ethyl acrylate (II:$R_3$=H) (18.4 ml, 0.17 mol) and the resulting mixture is stirred magnetically at 35°C for 48 hours. After evaporation of the solvent at reduced pressure, the residue is taken up with $Et_2O$ and the organic phase is washed 3 times with $H_2O$, dried over anhydrous $MgSO_4$ and evaporated at reduced pressure. A raw oil is obtained which by distillation at reduced pressure gives III ($R_1$=$C_6H_5$, $R_2$=$CH_3$,$R_3$=H) (23.0 g, 58% calculated on the ethyl acrylate): bp 112°-113°C/0.1 mmHg.

Example 8

Preparation of oximinopropionic acid (IV:$R_1$=$C_6H_5$,$R_2$=$CH_3$,$R_3$=H, compound 6).

To a solution of III ($R_1$=$C_6H_5$,$R_2$=$CH_3$,$R_3$=H) (16.5 g, 0.070

mol) in ethanol (13 ml) is added an ethanolic solution of 2N KOH (42 ml, 0.084 mol). The resulting mixture is left 48 hours at room temperature. After evaporation of the organic solvent at reduced pressure the solid residue is taken up with $H_2O$ and the aqueous phase is washed twice with $Et_2O$ and then acidified to pH 1-2 with dilute HCl and extracted 3 times with $Et_2O$.

The ethereal phase washed twice with $H_2O$ and dried over anhydrous $MgSO_4$ is evaporated at reduced pressure to give a raw solid (14.0 g) which by crystallization from petroleum ether gives compound 6 practically pure (11.9 g, 82%).

The compounds of the invention were tested to evaluate their antiinflammatory, analgesic, antipyretic, platelet aggregation inhibition and cytostatic pharmacological effects. At the same time acute toxicity tests were made in the mouse with evaluation of $LD_{50}$ and of the symptomatology resulting from the treatment.

Antiinflammatory action was evaluated in the growing male Wistar rat with respect to edema induced in the paw by injection of 0.1 ml of carrageenin in a 1% aqueous solution. The compounds were administered orally in the various dosages one hour before the edemogen agent and verification of the pharmacological effect was continued for 24 hours after injection of the edemogen agent.

Analgesic action was evaluated in the male Wistar rat with the Randall and Selitto method which consists of evaluating the pain threshold induced by the application of a constant load on the paw in which was induced a phlogosis by the injection of 0.1 ml of carrageenin in a 1 % aqueous solution. The compounds were administered orally in the various dosages one hour after administration of the edemogen agent and verification of the pharmacological effect was continued for four hours after administration of

the compounds.

Antipyretic action was evaluated in the growing male Wistar rat compared with hyperthermia induced by subcutaneous injection in the abdominal region of 20% beer yeast in aqueous solution in a quantity of 1 ml/kg of body weight.

The compounds were administered orally one hour after injection of the yeast and verification of the pharmacological effect was continued for four hours after administration of said products.

Inhibition of platelet aggregation was evaluated in vitro according to the Born method with various inducers (arachidonic acid, adrenalin, collagen) using as the substrate human plasma from healthy donors. For some new compounds the generation of thromboxane $B_2$ and 6-keto-PGF1 according to the De Fryen method was evaluated. Platelet aggregation was studied in two successive periods: immediately after addition of the derivative and after 15 minutes of incubation at 37°C.

Cytostatic action was tested on cellular lines of Fibrosarcoma 3T3-B77 obtained by conversion of 3T3 Bald/C fibroblasts by Bratislava 77 strain RSV virus and on MELANOMA B16-F1 cells. The influence of cellular proliferation induced by the addition in vitro of the compounds in different concentrations was evaluated.

$LD_{50}$ and toxic symptomatology resulting from the treatment were evaluated in the growing Swiss mouse by intraperitoneal administration of the different compounds. The period of observation was extended for 10 days after administration and the value of the lethal dose was calculated by graphic interpolation of the number of deaths.

<u>Results of pharmacological analyses</u>

In Table II are given acute toxicity data (LD50 calculated with

the graphic method) obtained in the mouse after intraperitoneal administration of the various compounds and the symptomatological observations attributable to the treatment.

TABLE II

| Product | LD50 after mg/kg ip | Symptomatology |
|---|---|---|
| 1 | 200 | agitation |
| 2 | 50 | agitation |
| 3 | 300 | passivity |
| 4 | 450 | passivity |
| 5 | 400 | agitation |
| 6 | 600 | passivity |
| 7 | 300 | passivity |
| 8 | 600 | passivity |
| 9 | 1000 | passivity |
| 10 | 600 | agitation |
| 11 | 250 | agitation |
| 12 | 850 | passivity |
| 13 | 250 | passivity |
| 14 | 350 | passivity |
| 15 | 1500 | negligible |
| 16 | 600 | passivity |
| 17 | 400 | passivity |
| 18 | 325 | passivity |
| 19 | 1800 | passivity |
| 20 | 650 | passivity |
| 21 | 525 | passivity |
| 22 | 1800 | passivity |
| 23 | 510 | passivity |
| 24 | 750 | passivity |
| 25 | 800 | passivity |

In Table III are given the data on antiinflammatory, analgesic and antipyretic action obtained with the abovementioned methods at a dosage selected as a function of $LD_{50}$ and symptomatology.

TABLE III

| Product | Dosage mg/kg ip | Antiinflammatory action | Analgesic action | Antipyretic action |
|---|---|---|---|---|
| | | (maximum % response in first 4 hours) | | |
| 1 | 20 | 30 | 35 | 15 |
| 2 | 5 | 10 | 15 | 10 |
| 3 | 25 | 18 | 25 | 0 |
| 4 | 25 | 15 | 20 | 10 |
| 5 | 25 | 42 | 45 | 30 |
| 6 | 25 | 26 | 30 | 15 |
| 7 | 10 | 14 | 10 | 10 |
| 8 | 40 | 70 | 85 | 40 |
| 9 | 50 | 38 | 45 | 25 |
| 10 | 30 | 9 | 10 | 0 |
| 11 | 10 | 10 | 15 | 0 |
| 12 | 30 | 23 | 30 | 20 |
| 13 | 50 | 0 | 0 | 0 |
| 14 | 30 | 18 | 25 | 25 |
| 15 | 60 | 17 | 20 | 10 |
| 16 | 60 | 11 | 20 | 0 |
| 17 | 50 | 34 | 40 | 30 |
| 18 | 35 | 36 | 45 | 25 |
| 19 | 100 | 10 | 20 | 15 |
| 20 | 60 | 25 | 35 | 20 |
| 21 | 55 | 21 | 20 | 20 |
| 22 | 100 | 18 | 25 | 20 |
| 23 | 50 | 40 | 55 | 50 |

| | | | | |
|---|---|---|---|---|
| 24 | 50 | 35 | 46 | 40 |
| 25 | 50 | 35 | 40 | 35 |

For compound no. 8 which from the preliminary data given in Table II seems to be more active the experiments for evaluation of antiinflammatory, analgesic and antipyretic action were researched in comparison with Indomethacin and Piroxicam.

The data obtained are given in Tables IV, V, and VI. Table VII recapitulates the comparative values of the two compounds.

TABLE IV

Antiinflammatory action - edema from carrageenin

| Product | Dosage mg/kg/os | Max % resp. (1st 4 h) | ED50 mg/kg/os |
|---|---|---|---|
| 8 | 3 | 7.4 | |
| | 10 | 29.1 | 33 |
| | 30 | 46.7 | |
| Indomethacin | 3 | 27.0 | |
| | 10 | 40.5 | 24 |
| | 30 | 52.6 | |
| Piroxicam | 1 | 15.6 | |
| | 3 | 43.1 | 4.6 |
| | 10 | 67.0 | |

TABLE V

Antiinflammatory action - hyperthermia from beer yeast

| Product | Dosage mg/kg/os | Max % resp. (1st 4 h) | ED50 mg/kg/os |
|---|---|---|---|
| 8 | 10 | 11 | |
| | 20 | 35.5 | 46 |
| | 60 | 52.7 | |
| Indomethacin | 3 | 20.2 | |
| | 10 | 50.9 | 9.8 |

| | | | | |
|---|---|---|---|---|
| | 30 | 74.2 | | |
| Piroxicam | 1 | 17.1 | | |
| | 3 | 43.7 | 3.6 | |
| | 10 | 75.4 | | |

TABLE VI

Analgesic action - Randall & Selitto test

(rise of pain threshold)

| Product | Dosage mg/kg/os | Sound limb % max variation | Inflamed limb % max variation | ED50 mg/kg/os |
|---|---|---|---|---|
| 8 | 3 | - | - | |
| | 10 | 11 | 40 | 12.5 |
| | 30 | 14 | 90 | |
| Indomethacin | 3 | - | 35 | |
| | 10 | 6 | 66 | 5.5 |
| | 30 | 14 | 94 | |
| Piroxicam | 1 | - | 20 | |
| | 3 | - | 49 | 3.0 |
| | 10 | 10 | 82 | |

TABLE VII

(recapitulation)

| Product | LD50 Mouse | | ACTION | | |
|---|---|---|---|---|---|
| | | | antiinflamatory | antipyretic | analgesic |
| | i.p. | os | ED50 mg/kg/os | ED50 mg/kg/os | ED50 mg/kg/os |
| 8 | 600 | 700 | 33 | 46 | 12.5 |
| Indomethacin | 125 | 160 | 24 | 9.8 | 5.5 |
| Piroxicam | 90 | 360 | 4.6 | 3.6 | 3.0 |

On some of the compounds which are the object of the present invention differentiated independently of the magnitude of the antiinflamatory, analgesic and antipyretic effect, preliminary tests in vitro were performed under the experimental conditions set forth above for the purpose of evaluating their effect on platelet reactivity.

The results obtained using as the antagonist arachidonic acid are given in Table VIII.

For compound 8, which proved to be among the more active, the data in comparison with other inducers i.e. adrenalin and collagen, are given in Table IX.

The results obtained on the generation of TXB2 and 6-Keto-$PGF_{12}$ evaluated to determine preliminarily the level of interaction on the biochemical mechanisms underlying increased platelet reactivity are given in Table X.

TABLE VIII

Platelet aggregation in vitro in accordance with Born; inducer: 1 mM of arachidonic acid.

| Product | Concentration | Percentage response variation | |
|---|---|---|---|
| | M | Lagt | AM |
| 8 | $5.10^{-4}$ | +114.3 | -32.3 |
| | $5.10^{-5}$ | +10.7 | -1.5 |
| 10 | $5.10^{-4}$ | +284.0 | -80.5 |
| | $5.10^{-5}$ | +17.9 | -12.5 |
| 16 | $5.10^{-4}$ | +28.0 | -13.3 |
| | $5.10^{-5}$ | -8.0 | -16.1 |
| 17 | $5.10^{-4}$ | +273.9 | -79.8 |
| | $5.10^{-5}$ | +60.0 | -33.3 |
| 22 | $5.10^{-4}$ | +276.0 | +80.5 |
| | $5.10^{-5}$ | +12.0 | +38.3 |

TABLE IX

Compound 8: platelet aggregation in vitro according to Born in comparison with collagen 4 µg/ml and adrenalin 10 µg/ml

COLLAGEN 4 µg/ml - without incubation

| Concentration M | Percentage response variation Lagt | AM |
|---|---|---|
| $5.10^{-4}$ | 3.2 | +0.3 |
| $5.10^{-5}$ | - | - |
| idem after incubation at 37°C for 15 min | | |
| $5.10^{-4}$ | +45.5 | -2.8 |
| $5.10^{-5}$ | +36.4 | -3.1 |

ADRENALIN 10 µg/ml without incubation

| | .2 WLT | AM |
|---|---|---|
| $5.10^{-4}$ | +75.0 | -19.5 |
| $5.10^{-5}$ | | |
| idem after incubation at 37°C for 15 min | | |
| $5.10^{-4}$ | +87.5 | -18.6 |
| $5.10^{-5}$ | +46.9 | -12.8 |

TABLE X

Compound 8 - Generation of TXB2 on whole blood after stimulation with collagen 1 µg/ml

| Concentration: | $5.10^{-5}$ M | $5.10^{-4}$ M |
|---|---|---|
| Basal: | 4.28 | 4.28 |
| 5 min | 54.3 (41.2) | 11.7 (41.2) |
| 15 min | 37.5 (26.5) | 8.6 (26.5) |
| 30 min | 41.4 (36.5) | 8.5 (36.5) |
| 60 min | 68 (65.8) | 15.1 (65.8) |

TXB2 values are expressed in µg/ml, control values in parentheses.

Compound 8 - Generation of 6-keto-PG $F_{12}$ on whole blood after

stimulation with collagen 1 μg/ml (preliminary tests)

| Concentration: | $5.10^{-5}$ M | $5.10^{-4}$ M |
|---|---|---|
| Basal: | 30 | 30 |
| 5 min | 256 (77) | 203.7 (78.75) |
| 15 min | 111.2 (37.5) | 35.5 (65) |
| 30 min | 153.5 (245.5) | 4.6 (306.6) |
| 60 min | 363.4 (160.2) | 162.4 (160.2) |

Values of 6-keto-PGF α are expressed in pg/ml.

Cytostatic action tested as described above on cellular lines of Fibrosarcoma 3T3-B77 and on cells of melanoma B16-Bratislava 77 made clear a significant influence on cellular proliferation, particularly evident for the compounds 1,2,8,9,13,23,24,25.

Cells incubated in the presence of the substances with concentrations of between $10^{-3}$ and $10^{-6}$ g/l at 37°C in a 5% $CO_2$ atmosphere at pH 7.4 in 10% of bovine fetal serum, demonstrate as mentioned above preliminarily a significant reduction in mitotic index velocity while not showing cytotoxic effects linkable to the concentrations used.

To preliminarily complete the pharmacological profile of the new series of compounds which are the object of the present invention and making reference to compound 8 a pharmacological test was performed in the rat and a subacute toxicity test was performed in the same animal species. The results of the haematic and urine rates obtained after oral treatment with 50 mg/kg os of compound 8 are given in Table XI. Determination was performed with HPLC of the content in biological liquids.

TABLE XI

Compound 8 - Analysis of absorption and elimination after administration of compound 8 in the rat at a dosage of 50 mg/kg.

| Hours of treatment | Plasma mcg/ml | Urine mcg/ml |
|---|---|---|
| 1 | 180 | |
| 2 | 145 | |
| 3 | 142 | |
| 4 | 141 | |
| 6 | 140 | |
| 8 | | 30 |
| 24 | 90 | 80 |
| 48 | 53 | 17 |

It is inferred from the results that the compound has a significantly long half-life (on the order of 24 hours).

Subacute toxicity assessed by repeated treatment for 30 days in the growing rat of both sexes with dosages up to 150 mg/kg/day revealed good tolerability of the compound, in particular as regards gastro-intestinal mucose where it is known that most nonsteroid antiinflamatory agents have ulcerogenic action. At the maximum dosages tested deaths occurring were accompanied by a significant reduction in the number of white corpuscles.

From this first set of analyses it can be summarized that the iminoether group in substitution of an aromatic ring in known nonsteroid antiinflammatory molecules makes it possible to retain the pharmacological properties of the antiinflammatory analogs.

It was ascertained that these compounds have excellent bioavailability (fast absorption) and, having a significantly long half-life, should be classified among long active substances.

Of particular importance concerning action on platelet hypere-activity it is noted that for the blockage of thromboxane production there is no corresponding blockage of prostacyclin production which on the contrary tends to increase. Blockage of

prostaglandins may be theorized on the basis of the nonsteroid antiinflammatory nature of the substances.

The pharmaceutical compositions which also form an object of the present invention are provided for administration both orally and parenterally.

For oral administration tablets and capsules are provided containing for a single daily administration from 100 to 1000 mg of active ingredient. This identifies the daily dosage.

For parenteral administration, since this is an acid substance, the corresponding nontoxic and pharmaceutically acceptable salts are preferred, preferably formed with weak bases such as carbonate, lysinate, et cetera.

The pharmaceutical compositions are prepared by the usual pharmaceutical techniques and with the usual excipients, vehicles and solvents.

CLAIMS

1. Derivatives of beta-oximino-propionic acid having the general formula

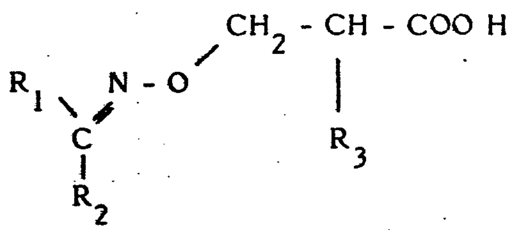

wherein $R_1$ and $R_2$ are hydrogen atoms or alkyl groups containing from 1 to 4 carbon atoms, straight or branched, or optionally substituted aryl or heterocyclic groups, or a butylene or pentylene group, and $R_3$ is hydrogen or an alkyl group containing 1 or 2 carbon atoms, and their pharmaceutically acceptable nontoxic salts.

2. Derivative in accordance with claim 1 characterized in that $R_1$ represents $C_6H_5$ and $R_2$=$R_3$=H.

3. Derivative in accordance with claim 1 characterized in that $R_1$=$R_3$=H and $R_2$=$C_6H_5$.

4. Derivative in accordance with claim 1 characterized in that $R_1$=$R_2$=$(CH_3)_2$-CH and $R_3$=H.

5. Derivative in accordance with claim 1 characterized in that $R_1$=$C_6H_5$,$R_2$=$CH_3$,$R_3$=H.

6. Process for preparation of the derivatives of claim 1 characterized in that an oxime of the following formula is reacted:

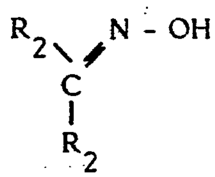

(I)

wherein $R_1$ and $R_2$ have the meanings indicated, with an alkyl acrylate of the formula:

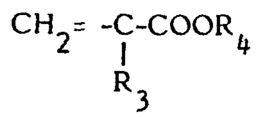

(II)

wherein $R_3$ has the meaning indicated above and $R_4$ is an alkyl, in the presence of a catalytic quantity of KOH, in an alcoholic solvent and at a temperature of between 35 and 60°C, giving the intermediate compound of the formula:

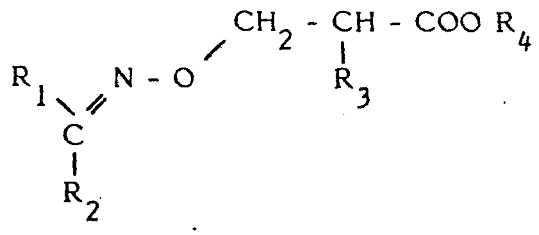

(III)

wherein $R_1$, $R_2$, $R_3$ and $R_4$ have the above meanings and compound III is saponified to give the corresponding acid, which is optionally salified with a nontoxic pharmaceutically acceptable base.

7. Process in accordance with claim 6 characterized in that said alkyl acrylate is ethyl acrylate.

8. Process in accordance with claim 6 characterized in that said alcoholic solvent is t-butanol.

9. Process in accordance with claim 6 characterized in that said saponification is performed with KOH.

10. Alkyl esters of beta-oximino-propionic acids having the formula:

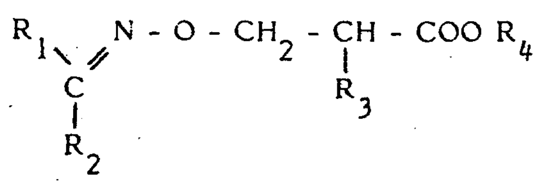

wherein $R_1$ and $R_2$ are hydrogen, a straight or branched alkyl group containing from 1 to 4 carbon atoms, an optionally substituted aryl or heterocyclic group or a butylene or pentylene group, $R_3$ is hydrogen or an alkyl group containing 1 or 2 carbon atoms,

and $R_4$ is an alkyl group.

11. Pharmaceutical composition characterized in that it contains as the active ingredient a derivative of beta-oximino-propionic acid in accordance with claim 1 together with the usual excipients and vehicles.

12. Pharmaceutical composition in accordance with claim 11 characterized in that said derivative comprising the active ingredient is the derivative set forth under claim 2.

13. Pharmaceutical composition in accordance with claim 11 and/or 12 with analgesic, antiinflammatory, antipyretic, platelet aggregation inhibiting and cytostatic activity.